# EUROPEAN PATENT APPLICATION

(11) **EP 4 825 364 A1**
(43) Date of publication of application: **21.10.2026**
(21) Application number: 25742323.6
(22) Date of filing: 17.01.2025
(51) Int. Cl.: A61K 8/29, A61K 8/81, A61K 8/73, A61K 8/85, A61K 8/39, A61Q 17/04

(54) **ULTRAVIOLET BLOCKING TITANIUM DIOXIDE COMPOSITE, AND ULTRAVIOLET BLOCKING TITANIUM DIOXIDE DISPERSION AND COSMETIC COMPOSITION THAT COMPRISE SAME**

(30) Priority: 18.01.2024 KR 20240007979
(71) Applicant: Chemland. Co., Ltd., Anseong-si, Gyeonggi-do 17602 (KR)
(72) Inventor: LEE, Beom Zoo, Seongnam-si, Gyeonggi-do 13544 (KR); LEE, Sul Hae, Gwacheon-si, Gyeonggi-do 13824 (KR); KANG, Kyung Kyu, Ansan-si, Gyeonggi-do 15475 (KR); HUH, Joon Hyuk, Pyeongtaek-si, Gyeonggi-do 17920 (KR)
(74) Representative: Isarpatent
(86) International application number: PCT/KR2025/099074
(87) International publication number: WO 2025/155175

(57) **Abstract**

The present invention relates to a titanium dioxide composite for ultraviolet (UV) protection, and a titanium dioxide dispersion and a cosmetic composition for UV protection including the same. According to an aspect of the present invention, the titanium dioxide composite for UV protection includes titanium dioxide and a polymer bound to a surface of the titanium dioxide, wherein the polymer includes an acrylate group at a terminal thereof.

## Description

### [Technical Field]

The present invention relates to a titanium dioxide composite for ultraviolet (UV) protection, and a titanium dioxide dispersion and a cosmetic composition for UV protection including the same. More particularly, the present invention relates to a titanium dioxide composite for UV protection having excellent UV-blocking performance, and a titanium dioxide dispersion and a cosmetic composition for UV protection including the same.

### [Background Art]

Ultraviolet (UV) protection cosmetics are products containing functional compositions that suppress skin aging (e.g., erythema, edema, freckles, or skin cancer) caused by UV rays by blocking such rays. These cosmetics are generally classified into inorganic UV protection cosmetics based on inorganic materials such as titanium dioxide and zinc oxide, and organic UV protection cosmetics based on organic materials such as ethylhexyl methoxycinnamate, ethylhexyl salicylate, octocrylene, ethylhexyl triazone, and benzophenone-3 (oxybenzone).

While inorganic UV protection cosmetics have the advantage of being less likely to cause skin irritation compared to organic UV protection cosmetics, they often result in a white cast (whitening effect) when applied to the skin and typically have a lower sun protection factor (SPF) than organic UV protection cosmetics.

Recently, active research has been conducted on inorganic UV protection compositions that maximize the UV blocking effect while minimizing the white cast by preparing titanium dioxide in the form of ultrafine particles. Particularly, by dispersing ultrafine rod-type titanium dioxide particles having a size of several nanometers to several hundred nanometers in an appropriate amount, transparency can be improved and UV protection efficiency can be maximized.

However, titanium dioxide has high photocatalytic activity. This photocatalytic action generates reactive oxygen species (ROS), which can cause other raw materials in the formulation to deteriorate or oxidize. Furthermore, ROS can attack the cell membranes and nuclei of skin cells, leading to DNA damage and skin aging.

To solve these problems, techniques for improving photocatalytic stability by surface-treating titanium dioxide to suppress its photocatalytic activity have been developed. Typically, techniques for modifying the surface of titanium dioxide with inorganic materials such as alumina and silica, or with fatty acids or silicone oil, have been introduced.

However, as titanium dioxide particles become ultrafine, it may be difficult to completely coat the surface of the particles. Conversely, when the particle diameter of titanium dioxide is increased, surface modification becomes easier, which can improve photocatalytic stability. However, this approach has the disadvantages of lowering the SPF or increasing the white cast.

Furthermore, if the surface of the titanium dioxide is not perfectly coated, electrons can be generated by photocatalytic action at the unmodified functional groups. These electrons can degrade the function of water-soluble thickeners used in oil-in-water (O/W) formulations, thereby impairing emulsion stability. In this case, the aggregation of titanium dioxide particles and water-soluble thickeners is induced, which can degrade the marketability of UV protection cosmetics formulated as an oil-in-water type.

To alleviate these problems, UV protection cosmetic compositions formulated as water-in-oil (W/O) or water-in-silicone (W/S) types may be used. However, these formulations tend to provide a heavy and sticky feeling of use and may cause skin irritation.

Accordingly, there is a demand for the development of a titanium dioxide dispersion and a cosmetic composition for UV protection that maintain an excellent SPF while simultaneously improving photocatalytic stability and exhibiting excellent formulation stability even in an oil-in-water formulation.

It should be noted that the aforementioned background art is technical information possessed by the inventors for the development of the present invention or acquired during the course of developing the present invention. Therefore, it cannot necessarily be regarded as prior art disclosed to the general public prior to the filing of the present application.

### [Disclosure]

### [Technical Problem]

An embodiment of the present invention is directed to providing a titanium dioxide composite for ultraviolet (UV) protection that maintains an excellent sun protection factor (SPF) while simultaneously exhibiting excellent photocatalytic stability, and a method of manufacturing the same.

An embodiment of the present invention is also directed to providing a cosmetic composition for UV protection that maintains an excellent SPF while simultaneously exhibiting excellent photocatalytic stability.

An embodiment of the present invention is also directed to providing a cosmetic composition for UV protection that does not cause functional degradation of a water-soluble thickener and exhibits excellent oil-in-water (O/W) formulation stability.

### [Technical Solution]

To achieve the aforementioned objects, according to an aspect of the present invention, a titanium dioxide composite for UV protection includes: titanium dioxide; and a polymer bound to a surface of the titanium dioxide, wherein the polymer includes an acrylate group at a terminal thereof.

According to another aspect of the present invention, the polymer may further include a beta-ketoester (β-ketoester) group capable of binding to the surface of the titanium dioxide.

According to still another aspect of the present invention, the polymer may further include at least one ester group in a main chain or side chain thereof.

According to yet another aspect of the present invention, the polymer may be formed by polymerization of different (meth)acrylate-based monomers.

According to yet another aspect of the present invention, the polymer may be formed from: a first monomer including any one selected from 2-(acetoacetoxy)methyl methacrylate, 2-(acetoacetoxy)ethyl acrylate, and 2-(acetoacetoxy)ethyl methacrylate; and a second monomer including any one selected from isobutyl methacrylate, butyl methacrylate, ethylhexyl acrylate, methyl methacrylate, butyl acrylate, and ethyl acrylate.

According to yet another aspect of the present invention, the first monomer and the second monomer may be polymerized in a ratio of 1:9 to 7:3.

According to yet another aspect of the present invention, the titanium dioxide composite may have a photocatalytic stability of 90% or more when measured according to the KS L ISO 10678 photocatalyst test method.

According to yet another aspect of the present invention, the titanium dioxide particles may have an average particle diameter of 10 to 100 nm.

According to yet another aspect of the present invention, the polymer may coat the titanium dioxide particles to a thickness of 10 nm or less.

According to yet another aspect of the present invention, the titanium dioxide particles may be coated with an inorganic material or an organic material.

To achieve the aforementioned objects, according to another aspect of the present invention, a titanium dioxide dispersion for UV protection includes: a titanium dioxide composite including titanium dioxide particles coated with a polymer including an acrylate group at a terminal thereof; and a dispersion stabilizer.

According to still another aspect of the present invention, at least a portion of a surface of the titanium dioxide particles may be coated with an inorganic material or organic material different from the polymer.

According to yet another aspect of the present invention, the dispersion stabilizer may include at least one selected from hydrogenated lecithin, cetearyl alcohol, behenyl alcohol, glyceryl stearate, polyhydroxystearic acid, PEG-10 dimethicone, and polyglyceryl-6 polyricinoleate.

To achieve the aforementioned objects, according to still another aspect of the present invention, a cosmetic composition for UV protection includes: an aqueous phase; a water-soluble thickener; an oil phase; and a titanium dioxide dispersion for UV protection, wherein the titanium dioxide dispersion for UV protection includes a titanium dioxide composite including titanium dioxide particles coated with a polymer including an acrylate group at a terminal thereof, and a dispersion stabilizer.

According to yet another aspect of the present invention, the water-soluble thickener may include at least one selected from carbomer, acrylates/C10-30 alkyl acrylate crosspolymer, hydroxyethylcellulose, hydroxypropyl methylcellulose, carboxymethylcellulose, ethylcellulose, xanthan gum, gellan gum, guar gum, and Arabic gum.

### [Advantageous Effects]

According to any one of the aforementioned technical solutions of the present invention, the titanium dioxide composite for UV protection, and the titanium dioxide dispersion and the cosmetic composition for UV protection including the same, are surface-modified with a polymer bound to the surface of the titanium dioxide, wherein the polymer includes an acrylate group at a terminal thereof. Accordingly, they can maintain an excellent sun protection factor (SPF) while simultaneously improving photocatalytic stability.

In addition, the cosmetic composition for UV protection of the present invention does not degrade the function of water-soluble thickeners. Consequently, when formulated as an oil-in-water (O/W) type, it exhibits excellent emulsion stability, thereby providing an outstanding feeling of use.

The effects obtainable in the present invention are not limited to the effects mentioned above, and other effects not mentioned herein will be clearly understood by those skilled in the art to which the present invention pertains from the following description.

### [Description of Drawings]

FIG. 1 is a flowchart illustrating a method of manufacturing a titanium dioxide composite for UV protection according to an embodiment of the present invention.
FIG. 2 is a graph showing the photocatalytic stability of titanium dioxide composites for UV protection according to embodiments of the present invention.
FIGS. 3A to 3H are graphs showing the UV blocking rates by wavelength of titanium dioxide composites for UV protection according to embodiments of the present invention and comparative examples.
FIG. 4 is a comparative graph illustrating the excellent photocatalytic stability of a titanium dioxide dispersion for UV protection according to an embodiment of the present invention.
FIG. 5 shows comparative photographs illustrating the excellent oil-in-water (O/W) formulation stability of a titanium dioxide dispersion for UV protection according to an embodiment of the present invention.
FIG. 6 shows comparative photographs illustrating the excellent stability over time of a titanium dioxide dispersion for UV protection according to an embodiment of the present invention.

### [Best Mode]

Hereinafter, embodiments of the present invention will be described in detail with reference to the accompanying drawings so that those skilled in the art to which the present invention pertains can easily implement the invention. However, the present invention can be embodied in various forms and thus is not limited to the embodiments described herein. In order to clearly explain the present invention in the drawings, parts unrelated to the description are omitted, and similar parts are given similar reference numerals throughout the specification.

Throughout the specification, when a part is referred to as being "connected" to another part, it includes not only the case where they are "directly connected" but also the case where they are "indirectly connected" with another member or element interposed therebetween. Moreover, when a part is described as "including" or "comprising" a certain component, it means that it may further include other components, without excluding other components unless specifically stated otherwise.

Hereinafter, the present invention will be described in detail with reference to the accompanying drawings.

The titanium dioxide composite for UV protection of the present invention includes titanium dioxide and a polymer bound to a surface of the titanium dioxide.

Titanium dioxide is a material formed by bonds between titanium atoms and oxygen atoms, and it has excellent reflectance against UVB and UVA.

The titanium dioxide may be used in any one phase among an anatase phase, a rutile phase, and a brookite phase, but preferably, the rutile phase may be used.

The titanium dioxide of the present invention may have an average particle diameter of 10 to 100 nm. In addition, the titanium dioxide of the present invention consists of rod-type particles and may have an average axial ratio of 1.2 to 5.0. The aforementioned titanium dioxide may be composed of the rod-type particles either individually or in a mutually aggregated form.

In some embodiments, the titanium dioxide may be coated particles. For example, the titanium dioxide may be a particle whose surface is coated with an inorganic material such as silicon oxide (SiOₓ) or aluminum oxide (AlₓO_{y}), or it may be a particle coated with an organic material such as stearic acid, glycerin, trimethoxycaprylylsilane, dimethicone, or simethicone. Preferably, the titanium dioxide may be a particle whose surface is coated with alumina (Al₂O₃).

The polymer of the present invention is a material for coating the surface of the titanium dioxide, and it may be a macromolecule formed through a polymerization reaction of two or more monomers.

The polymer includes a beta-ketoester (β-ketoester) group. The beta-ketoester functional group of the polymer is capable of forming a coordinate bond with the surface of the titanium dioxide. Specifically, a ketone functional group and an ester functional group of the beta-ketoester group form a coordinate bond with the surface of the titanium dioxide, thereby making it possible to uniformly coat the surface of the ultrafine titanium dioxide particles.

Furthermore, the polymer includes an acrylate group at a terminal thereof. Specifically, a (meth)acrylate functional group is located at the terminal of the polymer. The (meth)acrylate functional group at the terminal of the polymer may be formed by polymerization between a monomer including the aforementioned beta-ketoester group and a (meth)acrylate-based monomer.

In addition, the polymer includes at least one ester group. Here, the ester group may be generated during a process in which two or more monomers are mutually polymerized.

For example, the aforementioned polymer may be represented by the following Chemical Formula 1.

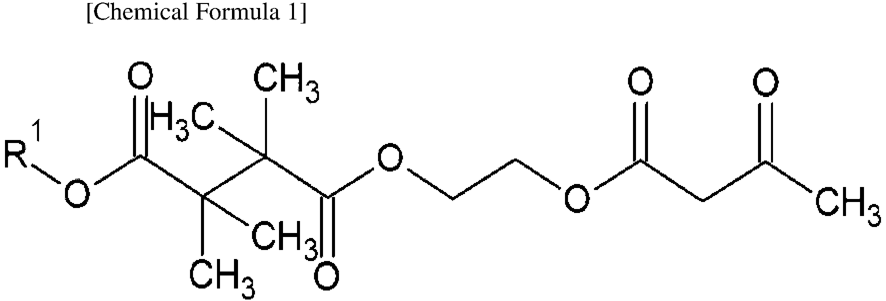

Here, R¹ may be an alkyl group having 1 to 10 carbon atoms.

The polymer may coat the surface of the titanium dioxide particles to a thickness of 10 nm or less. If the polymer is coated to a thickness exceeding 10 nm, the photocatalytic stability may be improved, but the large coating thickness may degrade the UV-blocking performance of the titanium dioxide.

The polymer of the present invention may be formed through a copolymerization reaction of two or more monomers. For example, the polymer may be formed through a copolymerization reaction of a first monomer and a second monomer.

The first monomer is a monomer having a beta-ketoester group and is involved in the surface modification of the titanium dioxide particles. The first monomer may be, for example, any one selected from 2-(acetoacetoxy)methyl methacrylate, 2-(acetoacetoxy)ethyl acrylate, and 2-(acetoacetoxy)ethyl methacrylate. Preferably, the first monomer may be 2-(acetoacetoxy)ethyl methacrylate (AAEMA).

The second monomer is a monomer that is polymerized with the first monomer to form an acrylate functional group at a terminal thereof, and it may be an acrylate-based monomer different from the first monomer. For example, the second monomer may be any one selected from isobutyl methacrylate, butyl methacrylate, ethylhexyl acrylate, methyl methacrylate, butyl acrylate, and ethyl acrylate. Preferably, the second monomer may be any one of isobutyl methacrylate, butyl methacrylate, ethylhexyl acrylate, and methyl methacrylate.

The first monomer and the second monomer may be polymerized in an appropriate ratio within a range of 1:9 to 7:3 (the ratio of the first monomer to the second monomer). When the titanium dioxide particles are surface-modified by the polymer formed within the aforementioned range, the titanium dioxide composite may maintain an excellent SPF. If the ratio of the first monomer to the second monomer exceeds 7:3, the SPF of the titanium dioxide composite may decrease, which may degrade its functionality as a UV protection cosmetic. Moreover, if the ratio of the first monomer to the second monomer is less than 1:9, the titanium dioxide particles may not be sufficiently surface-modified, which is undesirable because photocatalytic stability may be lowered.

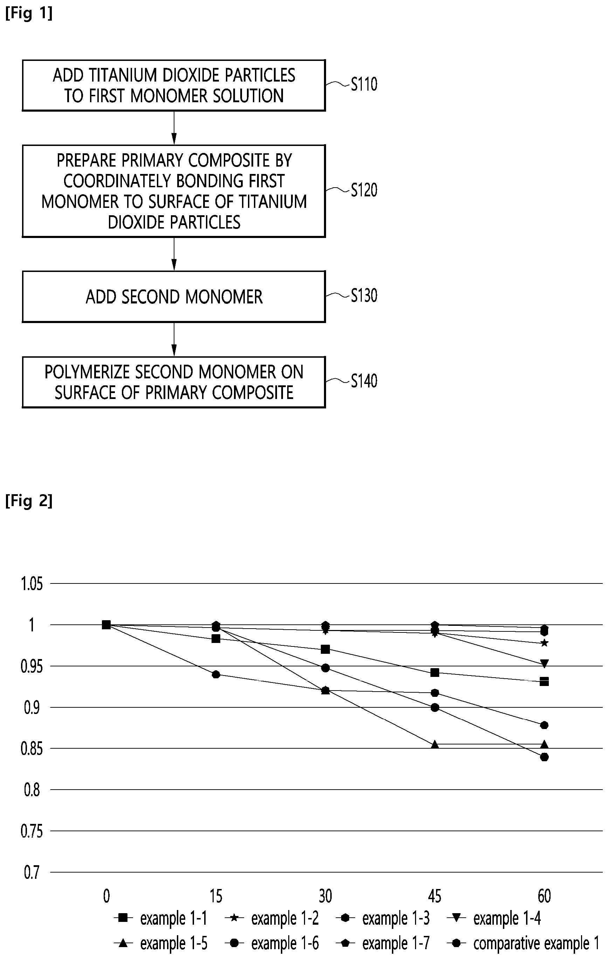

The surface modification of the titanium dioxide particles by the first monomer and the second monomer may proceed in an order in which the surface modification by the first monomer is induced by coordinate bonding via the beta-ketoester functional group of the first monomer, and then a copolymerization reaction between the second monomer and the terminal of the coordinated first monomer is induced. Reference is made to FIG. 1 for a detailed description thereof.

FIG. 1 is a flowchart illustrating a method of manufacturing a titanium dioxide composite for UV protection according to an embodiment of the present invention.

Referring to FIG. 1, the method of manufacturing a titanium dioxide composite for UV protection of the present invention begins by adding titanium dioxide particles into a first monomer solution (S110).

The first monomer solution may be prepared by dispersing a first monomer in a solvent.

As described above, an acrylate-based monomer having a beta-ketoester group may be used as the first monomer.

The solvent is a solvent capable of promoting coordinate bonding of the first monomer and is not particularly limited. For example, it may be a polar protic solvent, a non-polar solvent, or a mixed solvent thereof. As specific examples, it may be water, a glycol-based solvent, a glycol ether-based solvent, an alcohol-based solvent, a ketone-based solvent, an ester-based solvent, an amide-based solvent, a sulfoxide or sulfone-based solvent, a phenolic solvent, or a mixed solvent thereof. In this case, the glycol-based solvent may include ethylene glycol, propylene glycol, butylene glycol, triethylene glycol, hexylene glycol, polyethylene glycol, ethoxydiglycol, dipropylene glycol, or a combination thereof. The glycol ether-based solvent may include methyl glycol ether, ethyl glycol ether, isopropyl glycol ether, or a combination thereof. The alcohol-based solvent may include methanol, ethanol, n-propanol, isopropanol, butanol, or a combination thereof. The ketone-based solvent may include acetone, methyl ethyl ketone, methyl isobutyl ketone, or a combination thereof. The ester-based solvent may include ethyl acetate, butyl acetate, diethylene glycol ether acetate, methoxypropyl acetate, ethylene carbonate, propylene carbonate, or a combination thereof. The amide-based solvent may include dimethylformamide, dimethylacetamide, dimethyl caprylic/capric fatty acid amide, N-alkylpyrrolidone, or a combination thereof. The sulfoxide or sulfone-based solvent may include dimethyl sulfoxide (DMSO) or sulfolane, and the phenolic solvent may include toluene, xylene, and the like, but the present invention is not limited thereto. As a specific example, the organic solvent may be toluene, but is not limited thereto.

Thereafter, titanium dioxide particles are added to the first monomer solution.

In this case, the titanium dioxide particles may be added in a set amount such that the content of the first monomer is 50 to 500 parts by weight, preferably 100 to 300 parts by weight, based on 100 parts by weight of the titanium dioxide particles.

In addition, the titanium dioxide particles may be added such that the content of the solvent is 300 to 800 parts by weight, preferably 400 to 500 parts by weight, based on 100 parts by weight of the titanium dioxide particles.

By adding the titanium dioxide particles to the first monomer solution such that the contents of the first monomer and the solvent reach the aforementioned parts by weight based on the parts by weight of the titanium dioxide particles, the surface of the titanium dioxide particles may be uniformly modified by the first monomer.

Subsequently, in the method of manufacturing a titanium dioxide composite for UV protection of the present invention, a primary composite is manufactured by coordinately bonding the first monomer to the surface of the titanium dioxide particles (S120).

Specifically, by applying ultrasound after adding the titanium dioxide particles to the first monomer solution, coordinate bonding between the first monomer and the titanium dioxide particles may be induced. In this case, the ultrasound may be applied for a period ranging from 5 minutes to 30 minutes, and the process may be performed at room temperature. However, the process for inducing coordinate bonding between the first monomer and the titanium dioxide particles is not limited to the above example, and known processes capable of inducing coordinate bonding of the beta-ketoester group may be freely used.

Thereafter, in the method of manufacturing a titanium dioxide composite for UV protection of the present invention, an acrylate-based second monomer different from the first monomer is added (S130).

The second monomer is an acrylate-based monomer and, as described above, may be, for example, any one selected from isobutyl methacrylate, butyl methacrylate, ethylhexyl acrylate, methyl methacrylate, butyl acrylate, and ethyl acrylate.

The second monomer may be added alone into the mixed solution of the titanium dioxide particles and the first monomer, or it may be dispersed in a separate solvent and added in a solution form. In this case, the solvent into which the second monomer is added may be the same solvent as that of the aforementioned first monomer solution.

The addition of the second monomer may be performed such that the ratio of the first monomer to the second monomer is 1:9 to 7:3. The aforementioned ratio is the coating ratio of the first monomer and the second monomer, which may correspond to the concentration ratio of the first monomer and the second monomer.

Subsequently, in the method of manufacturing a titanium dioxide composite for UV protection of the present invention, the second monomer is polymerized on the surface of the primary composite (S140).

Specifically, as the second monomer is copolymerized with functional groups present on the surface of the primary composite, the surface of the primary composite is secondarily modified. As the second monomer modifies the surface of the primary composite, the titanium dioxide composite is obtained.

The copolymerization of the second monomer may be induced by a general copolymerization reaction of acrylate-based monomers, for example, the copolymerization reaction may be induced by heat or light. In this case, an initiator for initiating the copolymerization reaction may be added together during the addition of the second monomer.

As the surface of the primary composite is secondarily modified by the second monomer, the titanium dioxide composite includes a polymer coordinately bonded to the surface of the titanium dioxide inorganic particles via the beta-ketoester functional group. In this case, an acrylate functional group provided by the second monomer is present at the terminal of the polymer, and at least one ester functional group generated due to the copolymerization of the second monomer and the surface of the primary composite is present in a main chain or side chain of the polymer.

Meanwhile, in the titanium dioxide composite, the coating layer may have a thickness of 10 nm or less.

In some embodiments, after the copolymerization reaction of the second monomer is induced, a purification step may be further performed to obtain the titanium dioxide composite. Specifically, the titanium dioxide composite may be purified from the solvent through centrifugation. Thereafter, a titanium dioxide composite powder may be obtained through drying and pulverization.

The titanium dioxide composite of the present invention consists of ultrafine titanium dioxide particles having a size of 10 to 100 nm, and the surface of each titanium dioxide particle is uniformly modified by the polymer, thereby possessing excellent photocatalytic stability. In addition, the titanium dioxide composite undergoes a two-stage surface modification, specifically, it is secondarily modified with an acrylate-based second monomer different from the first monomer, so that an acrylate group may be located at the terminal of the polymer. Accordingly, the titanium dioxide composite of the present invention may maintain an excellent sun protection factor (SPF) while simultaneously having excellent photocatalytic stability.

The above-described titanium dioxide composite of the present invention may be dispersed in a solvent to be prepared in the form of a dispersion.

That is, the titanium dioxide dispersion of the present invention may include the titanium dioxide composite, a solvent, and a dispersion stabilizer.

The titanium dioxide composite is as described above, and redundant descriptions will be omitted.

The solvent may be an aqueous solvent including water or an oil-based solvent including oil.

The aqueous solvent may be, for example, water, a polar protic solvent, a ketone-based solvent, or a combination thereof. The polar protic solvent may be an alcohol-based solvent or a glycol-based solvent, and exemplarily, may be methanol, ethanol, propanol, butanol, ethylene glycol, propylene glycol, butylene glycol, polyethylene glycol (PEG), or a combination thereof, but is not limited thereto. The ketone-based solvent may exemplarily include acetone, methyl ethyl ketone (MEK), methyl isobutyl ketone (MIBK), or a combination thereof, but is not limited thereto.

The oil may be an oil used in food, medical compositions, or cosmetic compositions. For example, it may be a hydrocarbon-based oil, an ester-based oil, a vegetable oil, or a silicone-based oil, and although the oil is not particularly limited, a non-silicone-based oil may be suitable.

The dispersion stabilizer is a component for improving the dispersibility of the titanium dioxide composite in the solvent and minimizing the aggregation of the titanium dioxide composite. For example, it may include at least one of hydrogenated lecithin, cetearyl alcohol, behenyl alcohol, glyceryl stearate, polyhydroxystearic acid, PEG-10 dimethicone, and polyglyceryl-6 polyricinoleate. Preferably, it may include at least one of polyhydroxystearic acid and polyglyceryl-6 polyricinoleate.

The above-described dispersion stabilizer may be included in an amount of 5 wt% or less based on the total weight of the titanium dioxide dispersion. If the content of the dispersion stabilizer exceeds 5 wt%, dispersibility may be improved, but this is undesirable because the relative content of the titanium dioxide composite may be lowered as the content of the dispersion stabilizer unnecessarily increases.

The titanium dioxide dispersion of the present invention may be formulated into various forms, such as an oil-in-water (O/W) formulation and a water-in-oil (W/O) formulation. In particular, the titanium dioxide dispersion of the present invention may be formulated as an oil-in-water type and exhibits excellent formulation stability.

Specifically, the oil-in-water cosmetic composition of the present invention includes an aqueous phase, a water-soluble thickener, an oil phase, and a titanium dioxide dispersion for UV protection.

Since the titanium dioxide dispersion for UV protection has been described above, redundant descriptions will be omitted.

The aqueous phase is a solvent including water and may have the same composition as the above-described aqueous solvent.

The oil phase is a hydrophobic solvent including oil and may have the same composition as the above-described oil-based solvent.

The water-soluble thickener is a component that controls the viscosity of the cosmetic composition so that it has an appropriate viscosity, and allows the titanium dioxide composite to be dispersed within the formulation without settling. For example, it may include at least one of carbomer, acrylates/C10-30 alkyl acrylate crosspolymer, hydroxyethylcellulose, hydroxypropyl methylcellulose, carboxymethylcellulose, ethylcellulose, xanthan gum, gellan gum, guar gum, and Arabic gum.

The water-soluble thickener may be included in an amount optimized to achieve a specific viscosity of the cosmetic composition. For example, it may be included in an amount of 1 wt% or less based on the total weight of the cosmetic composition, but is not limited thereto.

In some embodiments, the cosmetic composition of the present invention may further include a neutralizing agent. The neutralizing agent is included to neutralize pH changes in the cosmetic composition, and tromethamine, triethanolamine (TEA), potassium hydroxide (KOH), sodium hydroxide (NaOH), or the like may be used, but is not limited thereto.

The titanium dioxide dispersion and the cosmetic composition including the same of the present invention include ultrafine titanium dioxide particles having a size of 10 to 100 nm, and the surface of each titanium dioxide particle is uniformly modified by a polymer, thereby having excellent photocatalytic stability. In addition, the above-described surface modification may be performed through a two-stage surface modification process. In this case, the titanium dioxide particles may be primarily surface-modified by a first monomer, and secondarily surface-modified by an acrylate-based second monomer different from the first monomer. Due to this, the titanium dioxide particles may be uniformly surface-modified with a polymer having an acrylate group at its terminal. The titanium dioxide dispersion and the cosmetic composition of the present invention, which include the titanium dioxide composite having these characteristics, may maintain an excellent sun protection factor (SPF) while simultaneously exhibiting excellent photocatalytic stability.

Furthermore, the titanium dioxide dispersion and the cosmetic composition of the present invention have almost no photocatalytic activity. By evenly modifying the surface of the titanium dioxide particles, the functional degradation of the water-soluble thickener may be minimized, thereby improving oil-in-water (O/W) formulation stability.

### [Modes of the Invention]

Hereinafter, the aforementioned advantages of the present invention will be described in detail through the following experimental examples.

### (Preparation Example 1: Preparation of titanium dioxide composite)

First, a titanium dioxide composite for UV protection was prepared according to the method of manufacturing a titanium dioxide composite for UV protection of the present invention.

Titanium dioxide composites according to the Preparation Examples were prepared by surface-modifying rod-type titanium dioxide powder (average particle diameter of 25 nm, average axial ratio of 3.5) with the materials shown in Table 1 below.

**[Table 1]**

| Classification | First monomer | Second monomer | Ratio (Note) |
|---|---|---|---|
| Preparation Example 1-1 | AAEMA (Acetoacetoxyethyl methacrylate) | IMA (Isobutyl methacrylate) | 5:5 |
| Preparation Example 1-2 | AAEMA | BMA (Butyl methacrylate) | 5:5 |
| Preparation Example 1-3 | AAEMA | EHA (Ethylhexyl acrylate) | 5:5 |
| Preparation Example 1-4 | AAEMA | MMA (Methyl methacrylate) | 5:5 |
| Preparation Example 1-5 | AAEMA | BA (Butyl acrylate) | 5:5 |
| Preparation Example 1-6 | AAEMA | EA (Ethyl acrylate) | 5:5 |
| Preparation Example 1-7 | AAEMA | - | 1:0 |
| Comparative Example 1 | Alumina | Stearic acid | Tayca Corporation |

Meanwhile, as Comparative Example 1, an MT100TV product manufactured by Tayca Corporation was obtained and used.

### (Experimental Example 1: Measurement of photocatalytic stability of titanium dioxide composite)

The photocatalytic stability of the titanium dioxide composites according to the above-described Preparation Examples 1-1 to 1-7 and Comparative Example 1 was measured.

The photocatalytic stability was evaluated according to the KS L ISO 10678 photocatalyst test method. Specifically, the method was carried out as follows: ① 0.02 g of methylene blue was added to 1 L of ethanol and stirred to prepare a 20 ppm methylene blue solution; ② 0.01 g of each of the titanium dioxide composites of Preparation Examples 1-1 to 1-7 and the titanium dioxide composite of Comparative Example 1, and 50 g of the methylene blue solution were placed in measuring cylinders and stirred, while standard samples were wrapped in foil to block UV rays; ③ a UV lamp was installed at the center of a multi-magnetic stirrer, and the measuring cylinders were placed at equal distances and stirred at 500 rpm for 1 hour; ④ test samples sufficient for absorbance measurement were taken, and the solid phase was separated from the liquid phase through centrifugation; ⑤ the absorbance of the standard samples and the test samples was measured at 655 nm using a spectrophotometer; and ⑥ the photocatalytic stability was calculated using Equation 1 below. [Equation 1] $Photocatalytic stability = \left({MB}_{UV}/{MB}_{NV}\right) \times 100$

Here, MB_{UV} is the absorbance of the test sample irradiated with UV after 1 hour of reaction, and MB_{NV} is the absorbance of the test sample not irradiated with UV after N hours of reaction.

The photocatalytic stability results measured by the above-described method are shown in FIG. 2.

FIG. 2 is a graph showing the photocatalytic stability of the titanium dioxide composites for UV protection according to embodiments of the present invention.

In addition, the result values of FIG. 2 are shown in Table 2 below.

**[Table 2]**

| | Time (min) | | | | | Photocatalytic stability (%) |
|---|---|---|---|---|---|---|
| | 0 | 15 | 30 | 45 | 60 | |
| Preparation Example 1-1 (AAEMA:IMA) | 1 | 0.984 | 0.972 | 0.942 | 0.932 | 93.20 |
| Preparation Example 1-2 (AAEMA:BMA) | 1 | 0.999 | 0.994 | 0.990 | 0.978 | 97.80 |
| Preparation Example 1-3 (AAEMA:EHA) | 1 | 0.999 | 0.994 | 0.994 | 0.992 | 99.20 |
| Preparation Example 1-4 (AAEMA:MMA) | 1 | 0.999 | 0.994 | 0.992 | 0.954 | 95.40 |
| Preparation Example 1-5 (AAEMA:BA) | 1 | 0.998 | 0.921 | 0.854 | 0.852 | 85.20 |
| Preparation Example 1-6 (AAEMA:EA) | 1 | 0.997 | 0.948 | 0.902 | 0.840 | 84.00 |
| Preparation Example 1-7 (AAEMA) | 1 | 0.999 | 0.998 | 0.997 | 0.997 | 99.70 |
| Comparative Example 1 (Alumina:Stearic acid) | 1 | 0.940 | 0.920 | 0.910 | 0.865 | 86.50 |

Referring to Table 2 and FIG. 2, it can be seen that the titanium dioxide composites of Preparation Examples 1-1 to 1-6, which are surface-modified with the first monomer and the second monomer, exhibit an excellent photocatalytic stability of 84% or more. Particularly, the titanium dioxide composites of Preparation Examples 1-1 to 1-4 exhibit an excellent photocatalytic stability of 90% or more, which is considered to be superior to the titanium dioxide composite of Comparative Example 1.

### (Experimental Example 2: Measurement of sun protection factor of titanium dioxide composite)

After diluting the titanium dioxide composites of Preparation Examples 1-1 to 1-7 and Comparative Example 1 described above in mineral oil, the sun protection factor (SPF) and the protection factor of UVA (PFA) were measured.

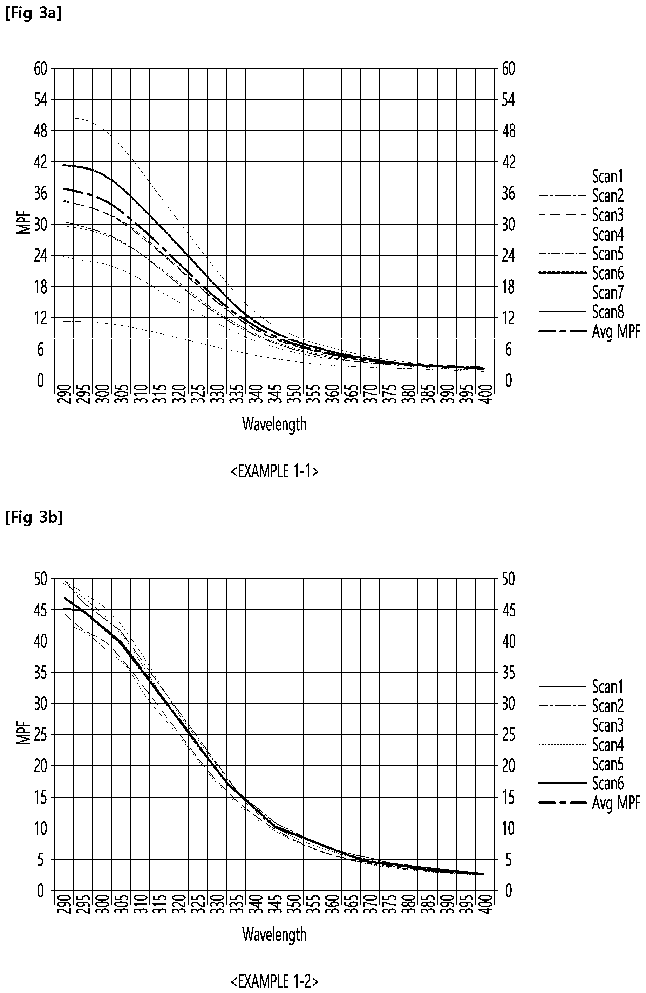

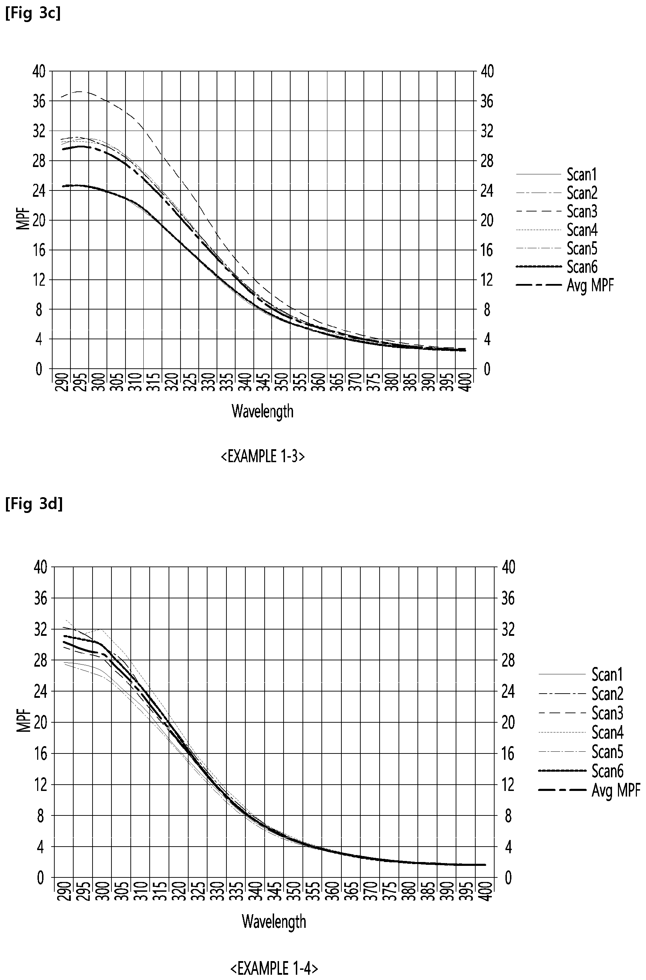

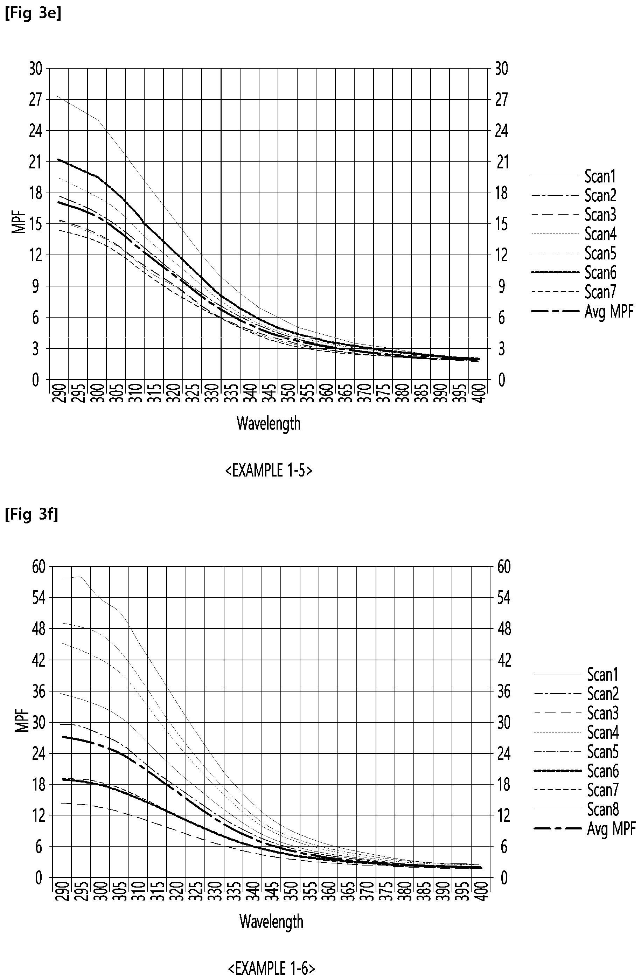

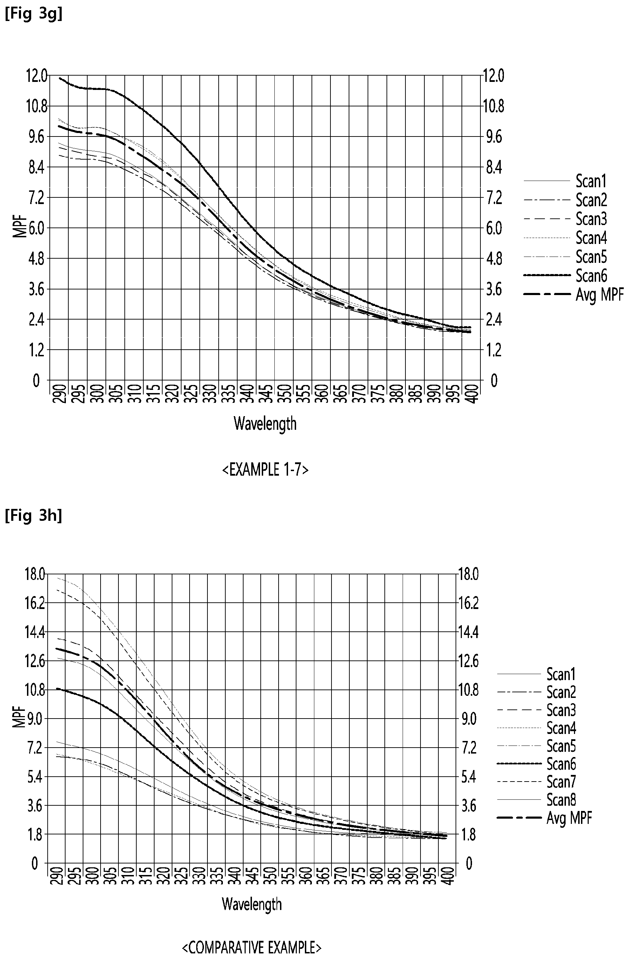

Specifically, the titanium dioxide composites of Preparation Examples 1-1 to 1-7 and Comparative Example 1 were diluted to a concentration of 8%. The SPF was measured according to ISO 24444, and the PFA was measured according to ISO 24442. The measurement results are shown in Table 3 below and FIGS. 3A to 3H.

FIGS. 3A to 3H are graphs showing the UV blocking rates by wavelength of the titanium dioxide composites for UV protection according to embodiments of the present invention and the comparative example.

**[Table 3]**

| Classification | SPF | PFA |
|---|---|---|
| Preparation Example 1-1 (AAEMA:IMA) | 20.56 | 7.72 |
| Preparation Example 1-2 (AAEMA:BMA) | 24.64 | 9.22 |
| Preparation Example 1-3 (AAEMA:EHA) | 19.69 | 8.01 |
| Preparation Example 1-4 (AAEMA:MMA) | 23.23 | 8.38 |
| Preparation Example 1-5 (AAEMA:BA) | 10.38 | 4.22 |
| Preparation Example 1-6 (AAEMA:EA) | 15.46 | 5.87 |
| Preparation Example 1-7 (AAEMA) | 7.92 | 4.21 |
| Comparative Example 1 (Alumina:Stearic acid) | 8.31 | 3.52 |

Referring to Table 3 and FIGS. 3A to 3H, it can be seen that the titanium dioxide composites of Preparation Examples 1-1 to 1-6 have a superior SPF and PFA compared to the titanium dioxide composite of Comparative Example 1. In particular, the titanium dioxide composites of Preparation Examples 1-1, 1-2, and 1-4 exhibit an SPF and PFA that are more than twice those of Comparative Example 1.

Meanwhile, it can also be seen that the titanium dioxide composites of Preparation Examples 1-1 to 1-6 have a superior SPF and PFA compared to Preparation Example 1-7. This is believed to be because aggregation between the titanium dioxide composites is suppressed and the uniformity of the surface modification is further increased by the secondary surface modification using the second monomer after the surface modification using the first monomer.

Furthermore, as illustrated in FIGS. 3A to 3H, the titanium dioxide composites of Preparation Examples 1-1 to 1-6 show relatively higher absorption capacities for UVA and UVB compared to Preparation Example 1-7 and Comparative Example 1. Particularly, the titanium dioxide composites of Preparation Examples 1-2 and 1-4 demonstrate an absorption capacity for UVA that is more than twice as high.

### (Preparation Example 2: Preparation of titanium dioxide composite)

Meanwhile, the present inventors prepared titanium dioxide composites as shown in Table 4 below to evaluate the degree of improvement in SPF and PFA according to the coating ratio of the first monomer and the second monomer of the titanium dioxide composite.

**[Table 4]**

| Classification | | TiO2 concentration (%) | First monomer concentration (%) | Second monomer concentration (%) | Coating ratio |
|---|---|---|---|---|---|
| AAEMA:IMA | Preparation Example 2-1 | 80 | 2 | 18 | 1:9 |
| | Preparation Example 2-2 | 80 | 6 | 14 | 3:7 |
| | Preparation Example 2-3 | 80 | 10 | 10 | 5:5 |
| | Preparation Example 2-4 | 80 | 14 | 6 | 7:3 |
| | Preparation Example 2-5 | 80 | 18 | 2 | 9:1 |
| AAEMA:BMA | Preparation Example 2-6 | 80 | 2 | 18 | 1:9 |
| | Preparation Example 2-7 | 80 | 6 | 14 | 3:7 |
| | Preparation Example 2-8 | 80 | 10 | 10 | 5:5 |
| | Preparation Example 2-9 | 80 | 14 | 6 | 7:3 |
| | Preparation Example 2-10 | 80 | 18 | 2 | 9:1 |
| AAEMA:EHA | Preparation Example 2-11 | 80 | 2 | 18 | 1:9 |
| | Preparation Example 2-12 | 80 | 6 | 14 | 3:7 |
| | Preparation Example 2-13 | 80 | 10 | 10 | 5:5 |
| | Preparation Example 2-14 | 80 | 14 | 6 | 7:3 |
| | Preparation Example 2-15 | 80 | 18 | 2 | 9:1 |
| AAEMA:MMA | Preparation Example 2-16 | 80 | 2 | 18 | 1:9 |
| | Preparation Example 2-17 | 80 | 6 | 14 | 3:7 |
| | Preparation Example 2-18 | 80 | 10 | 10 | 5:5 |
| | Preparation Example 2-19 | 80 | 14 | 6 | 7:3 |
| | Preparation Example 2-20 | 80 | 18 | 2 | 9:1 |

### (Experimental Example 3: Measurement of sun protection factor of titanium dioxide composite)

After diluting the titanium dioxide composites of Preparation Examples 2-1 to 2-20 described above in mineral oil, the sun protection factor (SPF) and the protection factor of UVA (PFA) were measured.

Specifically, the titanium dioxide composites of Preparation Examples 2-1 to 2-20 were diluted to a concentration of 8%. The SPF was measured according to ISO 24444, and the PFA was measured according to ISO 24442. The measurement results are shown in Table 5 below.

**[Table 5]**

| Classification | | SPF | PFA |
|---|---|---|---|
| AAEMA:IMA | Preparation Example 2-1 (9:1) | 3.48 | 1.74 |
| | Preparation Example 2-2 (7:3) | 19.42 | 6.05 |
| | Preparation Example 2-3 (5:5) | 20.56 | 7.72 |
| | Preparation Example 2-4 (3:7) | 20.73 | 8.88 |
| | Preparation Example 2-5 (1:9) | 24.52 | 9.05 |
| AAEMA:BMA | Preparation Example 2-6 (9:1) | 3.28 | 1.82 |
| | Preparation Example 2-7 (7:3) | 15.8 | 7.58 |
| | Preparation Example 2-8 (5:5) | 25.18 | 8.81 |
| | Preparation Example 2-9 (3:7) | 26.52 | 10.01 |
| | Preparation Example 2-10 (1:9) | 29.44 | 11.59 |
| AAEMA:EHA | Preparation Example 2-11 (9:1) | 4.83 | 1.54 |
| | Preparation Example 2-12 (7:3) | 11.42 | 6.84 |
| | Preparation Example 2-13 (5:5) | 16.67 | 8.01 |
| | Preparation Example 2-14 (3:7) | 21.44 | 8.91 |
| | Preparation Example 2-15 (1:9) | 26.72 | 9.81 |
| AAEMA:MMA | Preparation Example 2-16 (9:1) | 5.41 | 2.14 |
| | Preparation Example 2-17 (7:3) | 16.4 | 5.43 |
| | Preparation Example 2-18 (5:5) | 23.23 | 8.38 |
| | Preparation Example 2-19 (3:7) | 27.84 | 9.54 |
| | Preparation Example 2-20 (1:9) | 28.55 | 10.38 |

Referring to Table 5, the higher the concentration ratio of the second monomer, the higher the SPF and PFA. In particular, when the concentration ratio of the first monomer to the second monomer ranges from 1:9 to 7:3, the SPF is maintained at 15 or higher, and the PFA is also high, i.e.,7 or higher.

### (Preparation Example 3: Preparation of titanium dioxide dispersion)

To verify the excellent UV protection performance of the titanium dioxide dispersion prepared from the titanium dioxide composite of the present invention, a titanium dioxide dispersion was prepared by the following method.

First, a titanium dioxide composite was prepared in the same manner as in Preparation Example 2-18 described above.

Specifically, rod-type titanium dioxide powder (average particle diameter of 25 nm, average axial ratio of 3.5) was primarily surface-modified with AAEMA and secondarily surface-modified with MMA to prepare a titanium dioxide composite. In this case, the coating ratio of AAEMA:MMA was 5:5, and the purity of the titanium dioxide particles was 35%.

Thereafter, a dispersion was prepared by dispersing the titanium dioxide composite in a solvent with the composition shown in Table 6 below (Example 3). Specifically, C12-15 alkyl benzoate was used as a solvent, and polyhydroxystearic acid and polyglyceryl-6 polyricinoleate were used as dispersion stabilizers.

**[Table 6]**

| No. | Ingredients | Weight (%) |
|---|---|---|
| 1 | Titanium dioxide composite (AAEMA, MMA surface treated) | 50.0 |
| 2 | C12-15 alkyl benzoate | 46.5 |
| 3 | Polyhydroxystearic acid | 2.5 |
| 4 | Polyglyceryl-6 polyricinoleate | 1.0 |
| Total | | 100 |

Meanwhile, as comparative examples, dispersions of Comparative Examples 2 to 4 were prepared by dispersing a commercially available titanium dioxide composite (Comparative Example 1) with the compositions shown in Table 7 below.

For each dispersion, an appropriate solvent and dispersion stabilizer were selected according to the type of titanium dioxide composite.

**[Table 7]**

| | No. | Ingredients | Weight (%) |
|---|---|---|---|
| Comparative Example 2 | 1 | Titanium dioxide (alumina, stearic acid surface treated) | 50.0 |
| | 2 | Cyclopentasiloxane | 31.3 |
| | 3 | PEG-10 dimethicone | 8.2 |
| | 4 | Hexyl laurate | 10.0 |
| | Total | | 100 |
| | Titanium dioxide purity | | 40% |
| Comparative Example 3 | 1 | Titanium dioxide (alumina, hydrogen dimethicone surface treated) | 50 |
| | 2 | Cyclopentasiloxane | 31.7 |
| | 3 | PEG-10 dimethicone | 8.3 |
| | 4 | Hexyl laurate | 10 |
| | Total | | 100 |
| | Titanium dioxide purity | | 40% |
| Comparative Example 4 | 1 | Titanium dioxide (alumina, silica surface treated) | 52.5 |
| | 2 | Water | 45 |
| | 3 | Glycerin | 2 |
| | 4 | Caprylyl glycol | 0.5 |
| | Total | | 100 |
| | Titanium dioxide purity | | 37% |

### (Experimental Example 4: Measurement of photocatalytic stability of titanium dioxide dispersion)

The photocatalytic stability of the dispersions of Preparation Example 3 (Example 3) and Comparative Examples 2 to 4 described above was measured.

The photocatalytic stability was evaluated according to the KS L ISO 10678 photocatalyst test method, as described in Experimental Example 1 above.

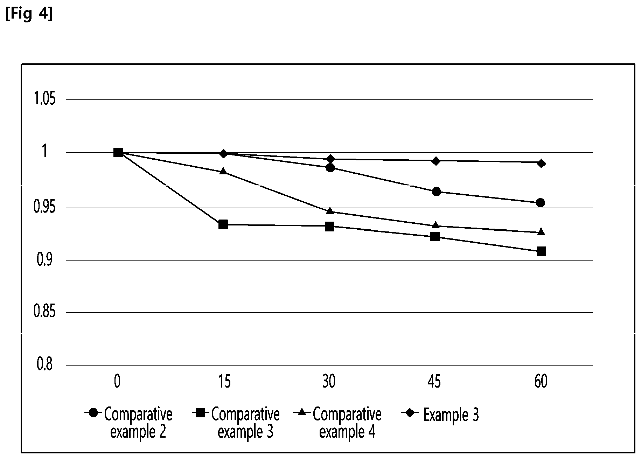

The photocatalytic stability results measured by the above-described method are shown in FIG. 4.

FIG. 4 is a comparative graph illustrating the excellent photocatalytic stability of the titanium dioxide dispersion for UV protection according to an embodiment of the present invention.

In addition, the result values of FIG. 4 are shown in Table 8 below.

**[Table 8]**

| | Time (min) | | | | | Photocatalytic stability |
|---|---|---|---|---|---|---|
| | 0 | 15 | 30 | 45 | 60 | |
| Comparative Example 2 | 1 | 0.999 | 0.987 | 0.964 | 0.952 | 0.952 |
| Comparative Example 3 | 1 | 0.933 | 0.931 | 0.921 | 0.908 | 0.908 |
| Comparative Example 4 | 1 | 0.982 | 0.945 | 0.931 | 0.925 | 0.925 |
| Example 3 | 1 | 0.999 | 0.994 | 0.992 | 0.990 | 0.990 |

Referring to Table 8 and FIG. 4, it can be seen that the titanium dioxide dispersion of the present invention (Preparation Example 3) has superior photocatalytic stability (99% or more) compared to the other dispersions (Comparative Examples 2 to 4). These results indicate that the surface modification of the titanium dioxide composite in the titanium dioxide dispersion of the present invention is almost perfectly achieved.

### (Experimental Example 5: Measurement of sun protection factor of titanium dioxide dispersion)

Preparation Examples 3-1 to 3-3 (Examples 3-1 to 3-3) were prepared by diluting the dispersion of Preparation Example 3 (Example 3) described above in the same solvent to concentrations of 10%, 20%, and 30%.

Similarly, Comparative Examples 2-1 to 4-3 were prepared by diluting each of the dispersions of Comparative Examples 2 to 4 in the same solvent as their respective solvents to concentrations of 10%, 20%, and 30%.

Thereafter, the sun protection factor (SPF) and the protection factor of UVA (PFA) were measured for Preparation Examples 3-1 to 3-3 and Comparative Examples 2-1 to 4-3. Specifically, the SPF was measured according to ISO 24444, and the PFA was measured according to ISO 24442.

In addition, based on Preparation Example 3-1 (Example 3-1), Comparative Example 2-1, Comparative Example 3-1, and Comparative Example 4-1 diluted to a concentration of 10%, the sun protection factor per 1% purity of titanium dioxide was calculated to comparatively analyze the SPF of the titanium dioxide composite for each sample.

The measurement results are shown in Table 9 below.

**[Table 9]**

| Classification | Concentration | SPF | PFA | SPF per 1% TiO2 |
|---|---|---|---|---|
| Preparation Example 3-1 | 10% | 6.90 | 2.80 | 1.97 |
| Preparation Example 3-2 | 20% | 23.31 | 7.87 | |
| Preparation Example 3-3 | 30% | 42.33 | 14.54 | |
| Comparative Example 2-1 | 10% | 6.23 | 2.85 | 1.02 |
| Comparative Example 2-2 | 20% | 11.94 | 4.78 | |
| Comparative Example 2-3 | 30% | 19.51 | 6.75 | |
| Comparative Example 3-1 | 10% | 4.06 | 1.94 | 1.57 |
| Comparative Example 3-2 | 20% | 8.38 | 3.19 | |
| Comparative Example 3-3 | 30% | 15.44 | 5.36 | |
| Comparative Example 4-1 | 10% | 5.71 | 2.95 | 1.54 |
| Comparative Example 4-2 | 20% | 12.5 | 6.05 | |
| Comparative Example 4-3 | 30% | 22.74 | 11.35 | |

Referring to Table 9, it can be seen that the titanium dioxide dispersions according to the embodiments of the present invention (Preparation Examples 3-1 to 3-3) exhibit a superior SPF and PFA compared to the titanium dioxide dispersions of the comparative examples (Comparative Examples 2-1 to 4-3). In particular, when used at a concentration of 20% or more, they exhibit nearly twice the SPF and PFA compared to the titanium dioxide dispersions of the comparative examples.

Furthermore, regarding the SPF value per 1% of the titanium dioxide composite, the titanium dioxide dispersion according to the example of the present invention (Preparation Example 3-1) is superior by at least 0.4 compared to the titanium dioxide dispersions of the comparative examples (Comparative Examples 2-1, 3-1, and 4-1).

This is believed to be because the titanium dioxide composites of the titanium dioxide dispersions according to the embodiments of the present invention undergo secondary surface modification by the second monomer after surface modification by the first monomer, thereby increasing the uniformity of the surface modification. Consequently, it is believed that aggregation between the titanium dioxide composites is suppressed, and the effect of UV reflection (or scattering) by titanium dioxide is maximized.

### (Preparation Example 4: Preparation of oil-in-water cosmetic composition for UV protection)

Meanwhile, to evaluate the oil-in-water (O/W) formulation stability of the titanium dioxide dispersion of the present invention, the present inventors prepared O/W cosmetic compositions for UV protection with the compositions shown in Table 10.

**[Table 10]**

| Classification | Ingredients | Composition 1 | Composition 2 | Composition 3 |
|---|---|---|---|---|
| Aqueous phase | Water | To 100 | To 100 | To 100 |
| | Butylene glycol | 0.5 | 0.5 | 0.5 |
| | Glycerin | 1.0 | 1.0 | 1.0 |
| | 1,2-Hexanediol | 2.0 | 2.0 | 2.0 |
| Water-soluble thickener | Carbomer | 0.2 | - | - |
| | Xanthan gum | - | 0.4 | - |
| | Hydroxyethyl cellulose | - | - | 0.4 |
| Oil phase | Polysorbate 60 | 0.7 | 0.7 | 0.7 |
| | Sorbitan sesquioleate | 0.5 | 0.5 | 0.5 |
| | Hydrogenated polydecene | 10.0 | 10.0 | 10.0 |
| | Dimethicone | 0.6 | 0.6 | 0.6 |
| | Caprylic/capric triglyceride | 10.0 | 10.0 | 10.0 |
| Dispersion | Dispersion of Example 3 to Comparative Example 4 | 20.0 | 20.0 | 20.0 |
| Neutralizing agent | Tromethamine | Appropriate amount | Appropriate amount | Appropriate amount |

For the dispersions in Table 10, the dispersion prepared in Preparation Example 3 (Example 3) described above and the dispersions of Comparative Examples 2 to 4 were used.

Referring to Table 10, carbomer was used as the water-soluble thickener in Composition 1, xanthan gum was used as the water-soluble thickener in Composition 2, and hydroxyethyl cellulose was used as the water-soluble thickener in Composition 3.

Therefore, a total of 12 types of cosmetic compositions were prepared by combining the 3 compositions, which differ by the type of water-soluble thickener, with the 4 dispersions.

### (Experimental Example 6: Evaluation of formulation stability)

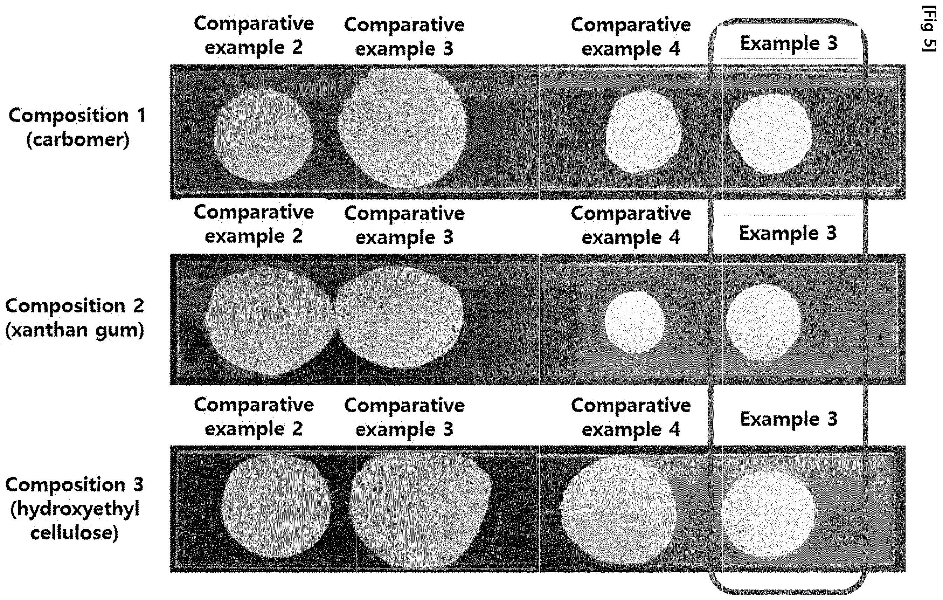

The emulsion stability of the 12 types of cosmetic compositions for UV protection of Preparation Example 4 described above was evaluated. The emulsion stability was evaluated by uniformly applying each cosmetic composition on glass and then visually observing whether aggregation occurred and whether water bled onto the surface of the applied material due to the separation of the aqueous phase and the oil phase. The results are shown in FIG. 5.

FIG. 5 shows comparative photographs illustrating the excellent oil-in-water formulation stability of the titanium dioxide dispersion for UV protection according to an embodiment of the present invention.

Referring to FIG. 5, the cosmetic compositions prepared using the titanium dioxide dispersion of the present invention (Preparation Example 3) showed no separation between the oil phase and the aqueous phase regardless of the type of water-soluble thickener, and maintained their applied shape, indicating excellent emulsion stability.

On the other hand, it was observed that when the water-soluble thickeners were used, the cosmetic compositions of Comparative Examples 2 to 4 exhibited aggregation and separation of the oil phase and the aqueous phase. In particular, in Composition 1 using carbomer as the water-soluble thickener and Composition 3 using hydroxyethyl cellulose as the water-soluble thickener, all dispersions of Comparative Examples 2 to 4 exhibited poor emulsion stability.

Meanwhile, in Composition 2 using xanthan gum as the water-soluble thickener, the dispersions of Comparative Example 4 and Preparation Example 3 (Example 3) exhibited relatively excellent emulsion stability. Therefore, the stability over time of Comparative Example 4 and Preparation Example 3 (Example 3) was additionally evaluated.

### (Experimental Example 7: Evaluation of stability over time)

As described above, the stability over time of the dispersion of Comparative Example 4 and the dispersion of Preparation Example 3 (Example 3) in Composition 2 using xanthan gum as the water-soluble thickener was evaluated.

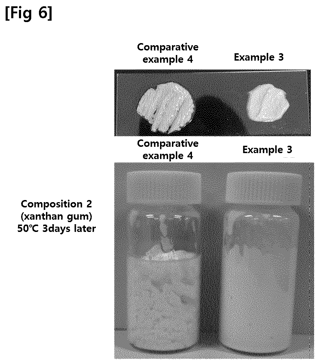

The stability over time was evaluated by exposing each cosmetic composition to a high-temperature environment of 50 °C for 3 days, and then visually evaluating its emulsion stability. The results are shown in FIG. 6.

FIG. 6 shows comparative photographs illustrating the excellent stability over time of a titanium dioxide dispersion for UV protection according to an embodiment of the present invention.

Referring to FIG. 6, the titanium dioxide dispersion of the present invention (Preparation Example 3) has superior stability over time compared to the dispersion of Comparative Example 4. That is, when exposed to a high-temperature environment of 50 °C for 3 days, the titanium dioxide dispersion of the present invention (Preparation Example 3) is applied evenly on the glass without aggregation, whereas the dispersion of Comparative Example 4 visibly undergoes aggregation and separation of the oil phase and the aqueous phase upon application.

This advantage is believed to be due to the fact that, in the titanium dioxide dispersion of the present invention, the titanium dioxide particles undergo a two-stage surface modification by different acrylate-based first monomer and second monomers, thereby improving the photocatalytic stability to 99% or more. In contrast, regarding the titanium dioxide dispersions according to the comparative examples, the surface modification is not uniform, so the generation of electrons is induced at the unmodified functional groups. It is believed that these generated electrons inhibit the function of the water-soluble thickener, thereby impairing the emulsion stability in the oil-in-water (O/W) formulation.

In addition, as verified in Experimental Example 5 above, the titanium dioxide dispersion of the present invention has the advantage of exhibiting excellent emulsion stability while simultaneously having an excellent sun protection factor (SPF). This is believed to be because, as described above, aggregation between the titanium dioxide composites is minimized due to the surface modification by different acrylate-based first monomer and second monomers, thereby maximizing the UV reflection effect by the titanium dioxide particles.

The above description of the present invention is for illustrative purposes, and those skilled in the art to which the present invention pertains will understand that it can be easily modified into other specific forms without changing the technical spirit or essential features of the present invention. Therefore, it should be understood that the embodiments described above are illustrative in all respects and not restrictive. For example, each component described as a single type may be implemented in a distributed manner, and similarly, components described as distributed may be implemented in a combined form.

The scope of the present invention is defined by the appended claims rather than the detailed description, and all changes or modifications derived from the meaning and scope of the claims and their equivalents should be construed as being included within the scope of the present invention.

### [Industrial Applicability]

The present invention can be applied to various daily necessity fields. For example, the present invention can be added to various daily necessities for skin and hair, such as cosmetics, soap, shampoo, and toothpaste, and can provide excellent UV protection performance.

## Claims

1. A titanium dioxide composite for ultraviolet (UV) protection, comprising:
titanium dioxide; and
a polymer bound to a surface of the titanium dioxide,
wherein the polymer includes an acrylate group at a terminal thereof.

2. The titanium dioxide composite of claim 1, wherein the polymer further includes a beta-ketoester (β-ketoester) group capable of bonding to the surface of the titanium dioxide.

3. The titanium dioxide composite of claim 2, wherein the polymer further includes at least one ester group in a main chain or side chain thereof.

4. The titanium dioxide composite of claim 1, wherein the polymer is formed by polymerization of different (meth)acrylate-based monomers.

5. The titanium dioxide composite of claim 4, wherein the polymer is formed from:
a first monomer including any one selected from 2-(acetoacetoxy)methyl methacrylate, 2-(acetoacetoxy)ethyl acrylate, and 2-(acetoacetoxy)ethyl methacrylate; and
a second monomer including any one selected from isobutyl methacrylate, butyl methacrylate, ethylhexyl acrylate, methyl methacrylate, butyl acrylate, and ethyl acrylate.

6. The titanium dioxide composite of claim 5, wherein the first monomer and the second monomer are polymerized in a ratio of 1:9 to 7:3.

7. The titanium dioxide composite of claim 1, wherein the polymer coats the titanium dioxide particles to a thickness of 10 nm or less.

8. The titanium dioxide composite of claim 1, wherein the titanium dioxide particles have an average particle diameter of 10 to 100 nm.

9. The titanium dioxide composite of claim 1, wherein the titanium dioxide composite has a photocatalytic stability of 90% or more when measured according to the KS L ISO 10678 photocatalyst test method.

10. The titanium dioxide composite of claim 1, wherein the titanium dioxide particles are coated with an inorganic material or an organic material.

11. A titanium dioxide dispersion for UV protection, comprising:
a titanium dioxide composite including titanium dioxide particles coated with a polymer including an acrylate group at a terminal thereof; and
a dispersion stabilizer.

12. The titanium dioxide dispersion of claim 11, wherein at least a portion of a surface of the titanium dioxide particles is coated with an inorganic material or organic material different from the polymer.

13. The titanium dioxide dispersion of claim 11, wherein the dispersion stabilizer includes at least one selected from hydrogenated lecithin, cetearyl alcohol, behenyl alcohol, glyceryl stearate, polyhydroxystearic acid, PEG-10 dimethicone, and polyglyceryl-6 polyricinoleate.

14. A cosmetic composition for UV protection, comprising:
an aqueous phase;
a water-soluble thickener;
an oil phase; and
a titanium dioxide dispersion for UV protection,
wherein the titanium dioxide dispersion for UV protection includes: a titanium dioxide composite including titanium dioxide particles coated with a polymer including an acrylate group at a terminal thereof; and a dispersion stabilizer.

15. The cosmetic composition of claim 14, wherein the water-soluble thickener includes at least one selected from carbomer, acrylates/C10-30 alkyl acrylate crosspolymer, hydroxyethylcellulose, hydroxypropyl methylcellulose, carboxymethylcellulose, ethylcellulose, xanthan gum, gellan gum, guar gum, and Arabic gum.
